(19)

Europäisches
Patentamt
European
Patent Office
Office européen
des brevets

(11) **EP 2 674 152 A1**

(12) **EUROPEAN PATENT APPLICATION**

(43) Date of publication:
**18.12.2013 Bulletin 2013/51**

(21) Application number: **13183542.3**

(22) Date of filing: **18.04.2011**

(51) Int Cl.:
*A61K 9/10* *(2006.01)*   *A61K 47/44* *(2006.01)*
*A61K 31/4439* *(2006.01)*   *A61K 31/401* *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.04.2010 DE 102010015143**

(62) Document number(s) of the earlier application(s) in
accordance with Art. 76 EPC:
**11731080.5 / 2 558 071**

(71) Applicant: **CTS Chemical Industries Ltd.
Kiryat Malachi 70953 (IL)**

(72) Inventor: **First, Sigal
47224 Ramat HaSharon (IL)**

(74) Representative: **Hiebl, Inge Elisabeth
Kraus & Weisert
Patent- und Rechtsanwälte
Thomas-Wimmer-Ring 15
80539 München (DE)**

Remarks:
This application was filed on 09-09-2013 as a
divisional application to the application mentioned
under INID code 62.

(54) **Stable liquid oily ready-to-use formulations, preparation thereof and use thereof**

(57)     The invention relates to a stable liquid oily ready-to-use formulation, comprising: (i) an active pharmaceutical ingredient, which has hydrophobic and/or lipophilic properties and/or which exhibits stability problems in aqueous environments, (ii) an oily vehicle, in which the active pharmaceutical ingredient is dissolved or dispersed, and which is selected from vegetable oils, synthetic oils, fatty acids or combinations thereof; and optionally one or more of a thickening/suspending agent, an antioxidant, a preservative, a flocculating agent, a surface stabilising agent, a sweetener, a flavouring agent, an emulsifier and a colouring agent, or combinations thereof, as well as to said formulation for use in the medical treatment of a patient group selected from (i) paediatric patients, (ii) elderly patients, (iii) patients suffering from dysphagia, or (iv) patients requiring medication via nasogastric or gastrostomy tubes and to a method for preparing a stable liquid oily ready-to-use formulation comprising the following steps: (a) heating the oily vehicle under mild stirring, (b) dissolving the antioxidant, (c) dissolving or melting the thickening/suspending agent and optionally emulsifying agent(s), until a clear solution is obtained, and cooling the solution to room temperature, (d) optionally adding further thickening/suspending agent, (e) adding and optionally disperging the active pharmaceutical ingredient, (f) optionally adding the sweetener, flavouring and/or colouring agents, (g) completing the volume with the oily vehicle to the desired amount, and (h) optionally performing homogenisation.

EP 2 674 152 A1

**Description**

The present invention relates to a stable liquid oily ready-to-use formulation comprising an active pharmaceutical ingredient, which has hydrophobic and/or lipophilic properties and/or which exhibits stability problems in aqueous environments. The present invention further relates to a method for preparing said formulation, and said formulation for use in the medical treatment of particular patient groups.

Many pharmaceutical ingredients (drugs) currently exist only in a solid dosage form such as tablets or granules. It would, however, often be advantageous to have a liquid dosage form of a drug at hand. A major problem concerning liquid formulations of a drug is the instability of many drugs in liquid media so that such formulations only have a very short shelf-life and not the desired shelf-life of 2 to 3 years.

Thus, not for all drugs suitable liquid dosage forms exist, in particular it is often difficult to provide a stable liquid dosage form of a drug.

A lack of commercially available oral liquid dosage forms is an ongoing problem in many practice settings. A pharmacist is often challenged to provide an extemporaneous oral liquid, for example for paediatric patients, patients who are unable to swallow solid dosage forms such as tablets or capsules, patients who must receive medications via nasogastric or gastrostomy tubes or patients who require non-standard doses that are more easily and accurately measured using a liquid formulation. It is common practice for this liquid dosage form to be prepared from a commercially available oral solid dosage form by simply crushing tablets or opening a capsule and the subsequent addition of water or juice. However, these dosage forms can become complex due to the addition of excipients and while these measures are taken to improve compliance and stability of the extemporaneously prepared product, there are often limited data to support the stability or bioavailability of the final liquid dosage form, where potential interactions between the vehicle, preservative, buffering agent, flavouring agent, levigating agent, suspending agent, viscosity enhancer, storage container and the modified commercial product have yet to be established.

The pharmacist, both in community and hospital pharmacy, is often challenged with the preparation of a liquid dosage form not available commercially for paediatric patients, those adults unable to swallow tablets or capsules. Appropriate formulations for administration to children exist for only a minority of commercially available drugs and the need for extemporaneously compounded formulations is escalating due to the release of many new drugs formulated for adults but with expected use in children. Children require titratable individualised doses in milligrams per kilogram of body weight and most children under six years of age cannot swallow tablets.

There are many reasons for the lack of commercially available paediatric formulations. The overall size of the paediatric market is much smaller than for adults, especially for common diseases such as hypertension. The industry is thus reluctant to commit resources to seek labelling for infants and children (unless a disease occurs exclusively or frequently in the paediatric population), since the formulation has to have been adequately studied in paediatric patients, It has been estimated that more than 40% of doses given in paediatric hospitals require compounding to prepare a suitable dosage form since crushing a tablet and/or sprinkling the contents of a capsule over food or mixing in a drink may lead to errors in preparation or delivery of doses.

Another practice seen in paediatric care is to use injectable solutions for oral administration. This is generally cost prohibitive and presents with many problems including the following:

(i) drugs and/or vehicles may be mucosal irritants, vesicants, nauseants, or cauterants;

(ii) drugs may undergo extensive first-pass metabolism or may have poor bioavailability after oral administration (e.g. cefuroxime and enalapril);

(iii) drugs and/or vehicles suitable for injection may be unpalatable;

(iv) excipients included in the formulation may have toxic effects when cumulative oral ingestion is considered; and

(v) co-solvents used in the commercial formulation may be diluted when mixed with syrup or water, thus allowing the drug to precipitate.

In most cases the pharmacist will therefore prepare an oral liquid dosage form with the active ingredient dissolved or suspended in a simple syrup or sorbitol mixture. Since pure crystalline powders of drugs are not usually accessible to pharmacies, the active pharmaceutical ingredient (API) is often obtained by modifying a commercially available adult solid dosage form by crushing a tablet or opening a capsule. When a drug is formulated for paediatrics use, several factors unique to paediatrics must be considered such as the immaturity of the intestinal tract and the subsequent influence on gastrointestinal absorption. Additives, including preservatives and sugar must be chosen carefully.

Formulations may also contain preservatives; an excipient considered to be largely inert in adults, however, may lead to life threatening toxicity in paediatrics when multiple doses of medications with the same preservative are employed. This is particularly the case with benzyl alcohol and benzoic acid. The physical, chemical, microbial and therapeutic stability of the above paediatric extemporaneous preparations may not have been checked at all. In addition, the increased potential for calculation or dispensing errors may prove the practice of modifying commercially available products to be extremely unsafe.

The stability of a pharmaceutical formulation is a major factor in ensuring the quality of the drug product and consequently, the efficacy of the treatment. Several parameters can influence the stability of a pharmaceutical formulation. These include the exposure of the product to a number of environmental conditions such as temperature, humidity and light. The chemical composition, the physical-chemical properties, the quantity of ingredients (both drug and excipients) in the formulation and the manufacturing process including storage and conditions during transportation may also affect the stability.

As an example, angiotensin-converting enzyme (ACE) inhibitors are widely used in the treatment of paediatric hypertension and have been found to be particularly effective treatments for hypertension in infants. ACE inhibitors are currently the principal agents for antihypertensive therapy in children both because of their effectiveness and their beneficial influence on cardiac and renal function and peripheral vasculature. However, drug administration to paediatric patients presents a number of challenges, since the pharmacist has to prepare an extemporaneous suspension by dispersing marketed tablets following their disintegration within liquids.

An enalapril suspension as a liquid dosage form was designed to address specific objectives such as ease and reproducibility of preparation for the pharmacy, ease of dosing, protection from microbial contamination, stability to support the suspension shelf-life, and acceptable taste for the patient (Rippley, R. K., Connor, J., Boyle, J., Bradstreet, T. E., Hand, E., Lo, M-W., Murphy, M. G., Biopharm. Drug Dispos. 2000, 21: 339-344). In this study, there were no adverse experiences related to formulation taste. However, this formulation is not a ready-to-use preparation, does not possess long term stability under standard storage conditions and has to be extemporaneously prepared by a pharmacist. Hence, there still is a need for a stable liquid ready-to-use dosage form of enalapril.

Despite many attempts to design a liquid dosage form of enalapril, up to now, there is no commercial ready-to-use liquid formulation of enalapril meeting health regulatory authorities requirements. This is probably due to the drug sensitivity in the liquid medium and lack of data on prolonged stability of the active compound in such medium over sufficient time to allow the design and the marketing of a viable commercial product.

It is therefore an object of the present invention to provide a pharmaceutical formulation that is suitable for the preparation of a ready-to-use liquid formulation. The ready-to-use formulation shall prevent the potential side effects that can be elicited by extemporaneous formulations which are not well characterised. The liquid formulation shall be suitable for the formulation of a wide variety of drugs and, in particular, for the formulation of drugs which have hydrophobic and/or lipophilic properties and/or exhibits stability problems in aqueous environment. Said formulation shall allow an accurate and precise dosing of the drug contained therein and shall be particularly suitable in the medical treatment of patient groups with swallowing problems such as paediatric or elderly patients. The drug shall be stable within said preparation for a long shelf-life.

The present invention is based on the unexpected and surprising finding that stable liquid formulations of drugs which have hydrophobic and/or lipophilic properties and/or exhibit stability problems in aqueous environments can be prepared using specific oily vehicles as a basis, in which the active pharmaceutical ingredient is dissolved or dispersed.

Thus, the present invention relates to a stable liquid oily ready-to-use formulation, comprising:

(i) an active pharmaceutical ingredient, which has hydrophobic and/or lipophilic properties and/or which exhibits stability problems in aqueous environments,

(ii) an oily vehicle, in which the active pharmaceutical ingredient is dissolved or dispersed, and which is selected from vegetable oils, synthetic oils, fatty acids or combinations thereof; and optionally one or more of a thickening/ suspending agent, an antioxidant, a preservative, a flocculating agent, an entero-coated polymer, a dipolar solvent (such as alcohol, glycerine etc.), a surface stabilising agent, a sweetener, a flavouring agent, an emulsifier, and a colouring agent, or combinations thereof.

The invention further relates to the stable liquid oily ready-to-use formulation for use in the medical treatment of a patient group selected from (i) paediatric patients, (ii) elderly patients, (iii) patients suffering from dysphagia, or (iv) patients requiring medication via nasogastric or gastrostromy tubes.

The invention further relates to a method for preparing said stable liquid oily ready-to-use formulations comprising the steps of

(a) heating the oily vehicle under mild stirring,

(b) dissolving the antioxidant,

(c) dissolving or melting the thickening/suspending agent, until a clear solution is obtained, and cooling the solution to room temperature,

(d) optionally adding further thickening/suspending agent,

(e) adding and optionally dispersing the active pharmaceutical ingredient,

(f) optionally adding the sweetener, flavouring and/or colouring agents,

(g) completing the volume with the oily vehicle to the desired amount, and

(h) optionally performing homogenisation.

The active pharmaceutical ingredient comprised in the formulation according to the invention is an active pharmaceutical ingredient which has hydrophobic and/or lipophilic properties and/or which exhibits stability problems in aqueous environments. Pharmaceutical ingredients which have hydrophobic and/or lipophilic properties are classified as class 2 according to the FDA BCS classification. A drug has hydrophobic properties if the LogP of the drug product is > 5 and has hydrophilic properties if the LogP is < 5. LogP measurements are common technology in the field of pharmaceutical ingredients. A person skilled in the art is well in a position to carry out a corresponding test on the basis of common textbooks. According to a preferred embodiment of the invention, the active pharmaceutical ingredient has a LogP > 5. Pharmaceutical ingredients which exhibit stability problems in aqueous environments are sensitive principles which can be degraded as a result of hydrolysis, pH or oxidation. Examples thereof are omeprazole or aspirin.

The formulation according to the present invention is particularly suitable for the following active ingredients:

Domperidone, enalapril maleate, famotidine, gabapentin, granisetron HCl, itraconazole, ketoconazole, lansoprazole, levodopa/carbidopa, losartan potassium, ondansetron HCl, amiodarone HCl, captopril, rifampicin, sildenafil citrate, dexamethasone, verapamil, spironolactone, tacrolimus, tramadol HCl, tramadol with paracetamol, baclofene, terbinafine HCl, spironolactone with hydrochlorothiazide.

Preferably, the active pharmaceutical ingredient is enalapril maleate or omeprazole. Optionally, the active pharmaceutical ingredient is used together with an emulsifier (emulgator). Preferably, enalapril maleate or omeprazole are used together with an emulsifier. Said active ingredients may optionally be combined with the above ingredients.

According to the invention, the oily vehicle is selected from vegetable oils, synthetic oils, fatty acids or combinations thereof.

According to a preferred embodiment of the invention, the oily vehicle is a vegetable oil selected from wheat germ oil, soybean oil, olive oil, arachis oil, corn oil, cottonseed oil, linseed oil, coconut oil, rapeseed oil, borage seed oil, apricot kernel oil, peanut oil, sunflower oil, sesame oil, safflower oil or sweet almond oil or combinations thereof.

According to a preferred embodiment of the invention, the oily vehicle is a synthetic selected from medium chain triglycerides (MCTs), propylene glycol dicaprylocaprate, glyceryl caprylate/caprate, glyceryl cocoate, butylene glycol dicaprylate/caprate, coco caprylate/caprate, glyceryl behenate, glyceril monolinoleate, glyceryl oleate, hydrogenated vegetable oils, refined vegetable oils, glyceryl laurate, glyceryl myristate or combinations thereof.

According to a preferred embodiment, the oily vehicle is a fatty acid selected from C8 to C22 fatty acids saturated and unsaturated, omega-3, omega-6 or omega-9 fatty acids. For example caprilic acid, capric acid, lauric acid, myristic acid, palmitic acid, palmitoleic acid, margaric acid, heptadecic acid, stearic acid, oleic acid, linoleic acid, alpha and gamma linolenic acid, arachidic acid, eicosenoic acid and its' derivates (EPA, PGs, etc.), arachidonic acid, behenic acid, decosaenoic acid or a derivates of thereof, or a combination thereof. Preferably, the oily vehicle is soybean oil or a MCT. Examples of MCTs are caproic acid (C6), caprylic acid (C8), capric acid (C10) and lauric acid (C12). MCTs are composed of a glycerol backbone and three of these fatty acids. The approximate ratios of these fatty acids in commercial MCT products derived from coconut oil is 2 (C6) : 55 (C8) : 42 (C10) : 1 (C12).

According to a preferred embodiment of the invention, a thickening/suspending agent is selected from polysaccharides such as alginates, carageenan, xantangum, acacia, tragacanth, pectin, locust bean gum, guar gum; clays, such as magnesium aluminium silicates (veegum), kaolin, bentonite, hectorite; aliphatic acids and stearic salts, such as aluminium monostearates; colloidal silicon dioxide; long chain alcohols such as cetostearyl alcohol, cetyl alcohol; waxes such as beeswax; long-chain diacylglycerols such as glyceryl distearate, or combinations thereof. More preferably, the thickening/suspending agent is beeswax, cetyl alcohol, glyceryl distearate or a combination thereof.

A thickening agent is an agent which increases the viscosity of the solution and subsequently reduce the sedimentation rate of the dispersed particles. A suspending agent promotes dispersion of the particles in the liquid medium without necessarily increasing the viscosity by preventing aggregation or close contact between the disperse particles. The precipitate forms loose flocks that can be easily re-dispersed and return to the initial homogeneity and particle size distribution. The suspending agent can be a structured vehicle that prevents close contact between the dispersed particle upon sedimentation by forming a sieve structure network able to entrap the particles and avoid direct contact between them. It should be kept in mind that the shorter the distance between particles, the stronger the attraction forces between them resulting in the formation of aggregates that cannot be anymore separated into individual particles.

According to a preferred embodiment of the invention, the antioxidant may be selected from propyl gallate, octyl gallate, dodecyl gallate, butylated hydroxytoluene (BHT), butylated hydroxyanisole (BHA), tocopherol, D-alpha-tocopherol, DL-alpha-tocopherol, tocopheryl acetate, D-alpha- tocopheryl acetate, DL-alpha-tocopheryl acetate, diferoxamine mesylate, dilauryl thiodipropionate, or combinations thereof. The preservative may be selected from propyl gallate, butylated hydroxytoluene (BHT), butylated hydroxyanisole (BHA), D-alpha-tocopherol, dilauryl thiodipropionate or a combination of thereof.

An antioxidant is a substance capable of inhibiting oxidation, which may be added for this purpose to pharmaceutical products subject to deterioration by oxidative processes, as for example the development of rancidity in oils and fats or the inactivation of some medicinal in the environment of their dosage forms (Remington 21th ed.). A preservative is, in common pharmaceutical sense, a substance that prevents or inhibits microbial growth, which may be added to pharmaceutical preparations for this purpose to avoid consequent spoilage of the preparations by microorganisms (Remington 21th ed.). Substances of both categories may be added to the formulations of the present invention.

Preferably, the antioxidant/preservative is BHT and/or propyl gallate.

Suitable surface stabilising agents may be selected from kaolin, magnesium aluminium silicates, glycerine, bentonite, dimethicone and pectin or combinations thereof.

The formulation may optionally further comprise one or more emulsifier(s). Examples thereof are polysorbate 20, polysorbate 80, linoleoyl macrogolglycerides, lauroyl macrogolglycerides, oleoyl macrogolglycerides (polyoxylglycerides.

The ratio of the active pharmaceutical ingredients and the oily vehicle ranges from 0.1% to 20% preferably from 0.5% to 10%, more preferably from 1% to 10%. The further ingredients of the claimed ready-to-use formulation may be present in an amount from 0.1 % to 99.9%.

A preferred formulation of the invention comprises an active pharmaceutical ingredient selected from enalapril maleate (1%) or omeprazole (10%), an oily vehicle (90-99%) selected from soybean oil or medium chain triglyceride, thickening/suspending agent selected from beeswax (5%) or glyceride palmitostearate (3%), a preservative being propyl gallate (0.01%) and a surface stabilising agent being bentonite (6.5%).

The formulations according to the present invention will be used for the medical indication as given by the respective active pharmaceutical ingredient. A dosage will be determined by a physician in relation to the active ingredient and in relation to the kind and severity of the condition to be treated, the age and weight of the patient.

The formulation according to the present invention is particularly suitable for the medical treatment of patient groups having problems in swallowing a drug. Patients who are unable to swallow a drug are paediatric patients, elderly patients, patients suffering from dysphagia or patients requiring medication via nasogastric or gastrostromy tubes. The invention thus also relates to a method of treatment of said patients with the formulation according to the present invention.

According to a preferred embodiment of the invention, the formulation may be in the form of an oral, topical or parenteral formulation.

According to a preferred embodiment of the invention, the stable liquid oily ready-to-use formulations are used for the manufacture of a medicament for the medical treatment of a patient group selected from (i) paediatric patients, (ii) elderly patients, (iii) patients suffering from dysphagia, or (iv) patients requiring medication via nasogastric or gastrostomy tubes.

The formulation according to the present invention may basically be prepared according to methods generally known in the art. According to a preferred embodiment, said method comprises:

(a) heating the oily vehicle under mild stirring (50°C - 80°C)

(b) dissolving the antioxidant,

(c) dissolving or melting the thickening/suspending agent, until a clear solution is obtained, and cooling the solution to room temperature,

(d) optionally adding further thickening/suspending agent,

(e) adding and optionally dispersing the active pharmaceutical ingredient,

(f) optionally adding the sweetener, flavouring and/or colouring agents,

(g) completing the volume with the oily vehicle to the desired amount, and

(h) optionally performing homogenisation.

The method of preparing the formulation is according to general methods of the art (Remington 21th ed; Drugs and the pharmaceutical sciences textbook, Vol. 105 Pharmaceutical emulsions and suspensions, ed. by James Swarbrick 2000; The pharmaceutical codex 12th ed. Walter Lund 1994).

In a preferred embodiment, the active pharmaceutical ingredient may be granulated or encapsulated into microparticles prior to adding/dispersing the active pharmaceutical ingredient in the vehicle. In the case of pH sensitive molecules in the acidic range, then it will be worthy to protect the particles from degradation by coating them with an entero-coated polymer.

The liquid oily ready-to-use formulations according to the present invention have the advantage that they are ready-to-use formulations, i.e. they may easily be dosed for application to people having a disease which makes swallowing difficult or to children and elderly who cannot easily swallow solid dosage forms. Thus, an extemporaneous compounding is not required, and calculation or dispensing errors, which involve risks in the practice of modifying commercially available products are eliminated.

The enclosed Figures illustrate the subject-matter of the invention:

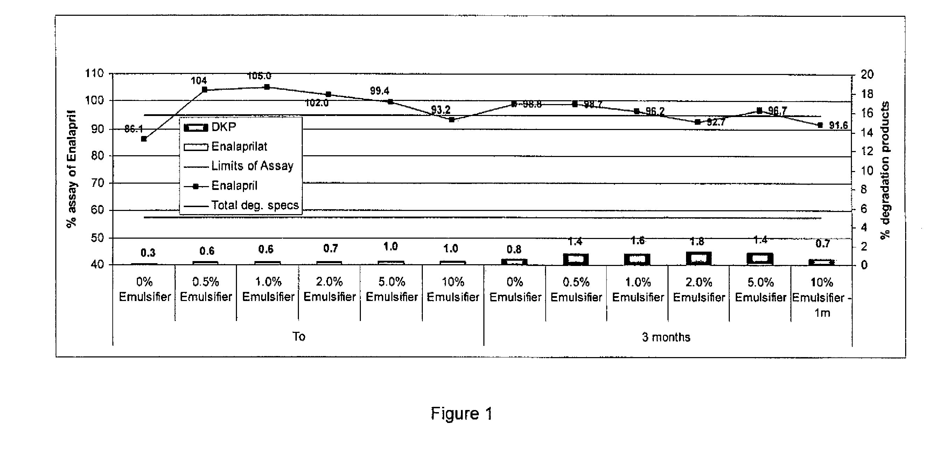

Figure 1 shows the results of the stability test with enalapril suspensions carried out without or with different concentrations of emulsifiers at 30°C/65% RH for 1 to 3 months (see Example 9).

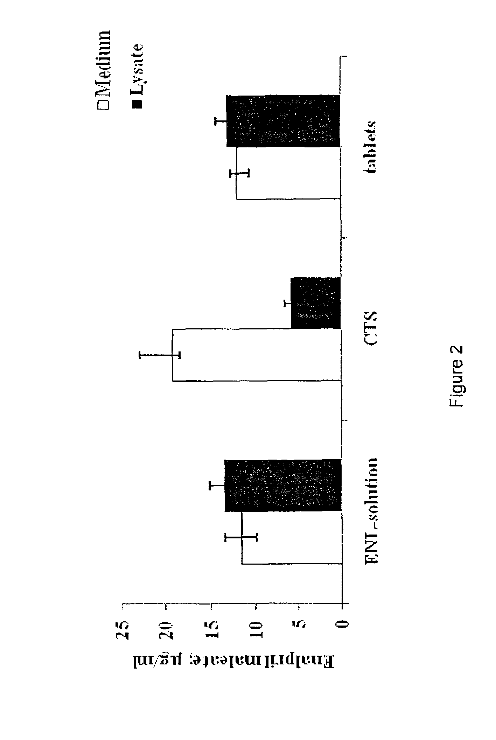

Figure 2 shows the distribution of enalapril maleate between medium and lysate (Example 11). Enalapril residue in Hanks buffer (pH 7.4) following 180 min incubation in Caco-2 cell monolayer and respective enalapril uptake following washing and cell lysis (SDS 0.2%) of various formulations containing 25 μg/ml enalapril maleate. N=4 in all the experiments. (ENL= Enalapril maleate; CTS= CTS oil formulation)

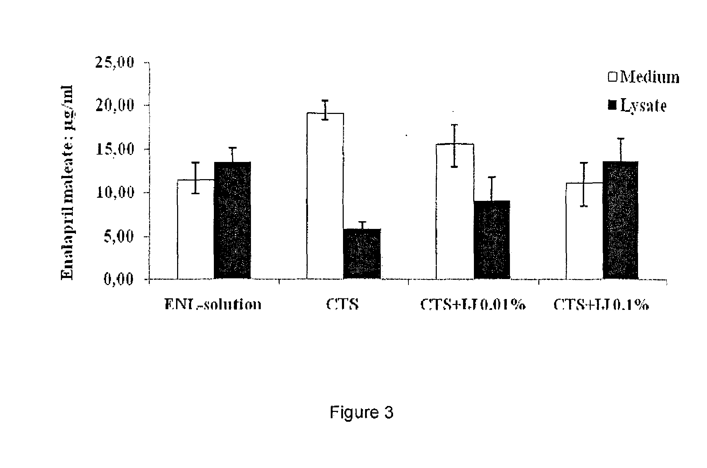

Figure 3 shows the enalapril uptake by the Caco-2 cell monolayer in the presence of increasing concentrations of rat intestinal juice. Enalapril residue in Hanks buffer (pH 7.4) and respective enalapril cell uptake following 180 min incubation in Caco-2 cell monolayer as a function of intestinal juice concentration, washing and cell lysis (SDS 0.2%) of CTS oil formulation containing 25 μg/ml enalapril maleate. N=4 in all the experiments. (ENL= Enalapril maleate; CTS= CTS oil formulation)

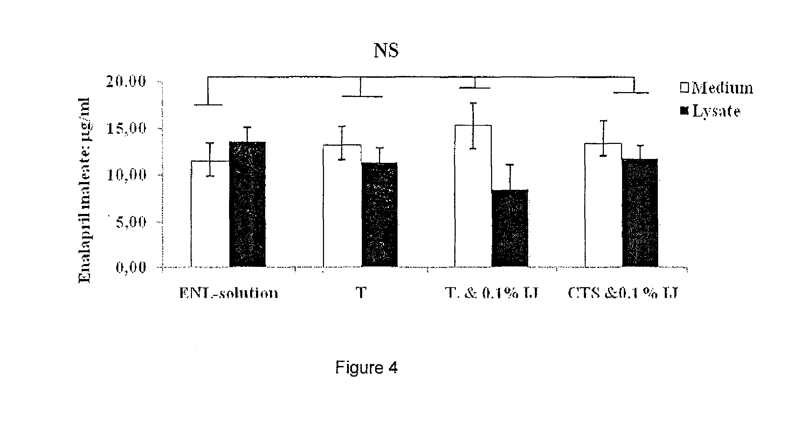

Figure 4 shows enalapril maleate residue in Hanks buffer (pH 7.4) medium following 3 h incubation in Caco-2 cell monolayer and respective enalapril maleate uptake following wash and cell lysis (SDS 0.2%) of selected formulations containing 25 μg/ml enalapril maleate. N=4 in all the experiments. The P value > 0.05, non-significant (NS). (ENL= Enalapril maleate; CTS= CTS oil formulation; IJ= intestinal juice; T= tablets)

The following examples illustrate further embodiments of the invention:

## Examples

## Examples 1 to 7

Method of preparation

In the following a general flowchart of the manufacturing process according to the present invention is given.

The following compositions were prepared according to the following method:

(a) heating the oily vehicle under mild stirring (50°C - 80°C)

(b) dissolving the antioxidant,

(c) dissolving or melting the thickening/suspending agent and optionally emulsifying agent(s), until a clear solution is obtained, and cooling the solution to room temperature,

(d) optionally adding further thickening/suspending agent,

(e) adding and optionally disperging the active pharmaceutical ingredient,

(f) optionally adding the sweetener, flavouring and/or colouring agents,

(g) completing the volume with the oily vehicle to the desired amount, and

(h) optionally performing homogenisation.

The optional emulsifying agent may be polysorbate 20, polysorbate 80, linoleoyl macrogolglycerides, lauroyl macrogolglycerides.

The stability of the formulations was checked according to USP/EP pharmacopeial specifications. Assay and Related substances, and Dissolution were measured by HPLC validated method based on EP and USP.

Any of the following compositions may contain flavouring and/or sweetening and/or colouring agents.

All amounts given in the examples are % w/w.

The following method example details an exemplary method of preparing the formulation of the invention further.

Method example

The oily vehicle is heated to 65- 70°C in water bath under mild stirring. The antioxidant/preservative (e.g. propyl gallate) is added. Then, a thickening/suspending agent, such as glyceryl palmitostearate or beeswax is dissolved or melted until a clear solution is obtained. The mixture is cooled down to the room temperature while stirring. Around 35°C, silica dioxide and bentonite (if used) are added, followed by the active pharmaceutical ingredient. At this point the sweetener, flavour and/or colour may be added. The volume is then completed with the oily base (MCT or soybean oil). Optionally, homogenisation is performed at the end.

During the entire procedure, it is important to maintain constant stirring in order to get the homogenous thickening effect and homogenous distribution of the active pharmaceutical ingredient.

Example 1

| | |
|---|---|
| enalapril maleate | 1% |
| glyceryl distearate | 3% |
| propyl gallate | 0.02% |
| soybean oil | ad 100% |

Example 2

| | |
|---|---|
| enalapril maleate | 1% |
| silica dioxide | 2-5% |
| glyceryl distearate | 3% |
| propyl gallate | 0.01% |
| MCT | ad 100% |

Example 3

| | |
|---|---|
| omeprazole Mg | 10% |
| silica dioxide | 2-6% |
| bentonite | 6.5% |
| propyl gallate | 0.01% |
| MCT | ad 100% |

Example 4

| amiodarone HCl | 10% |
|---|---|
| silica dioxide | 2-5% |
| glyceryl distearate | 3% |
| propyl gallate | 0.01% |
| MCT or soybean oil | ad 100% |

Example 5

| bisacodyl | 1% |
|---|---|
| glyceryl distearate | 3% |
| propyl gallate | 0.02% |
| soybean oil | ad 100% |

Example 6

| enalapril maleate | 1% |
|---|---|
| glyceryl distearate | 3% |
| sweetener | 0.1% |
| flavour | 0.2% |
| antioxidant (propyl gallate) | 0.02% |
| soybean oil | ad 100% |

Example 7

| amiodarone HCl | 10% |
|---|---|
| glyceryl distearate | 3% |
| Span 60 | 1.03%-2.55% |
| Tween 80 | 0.97%-2.45% |
| propyl gallate | 0.02% |
| aerosil | 0-2% |
| sweetener | 0.1 % |
| flavour | 0.2% |
| medium chain triglycerides | ad 100% |

Comparative Example 1

| Enalapril maleate | 1% |
|---|---|
| cetyl alcohol | 7% |
| propyl gallate | 0.02% |
| soybean oil | ad 100% |

Comparative Example 2

| enalapril maleate | 1% |
|---|---|
| silica dioxide | 2-5% |
| cetyl alcohol | 7% |
| propyl gallate | 0.01 % |
| MCT | ad 100% |

Comparative Example 3

| entero-coated omeprazole Mg | 5% |
|---|---|
| beeswax | 2-6% |
| bentonite | 6.5% |
| propyl gallate | 0.01% |
| MCT | ad 100% |

Comparative Example 4

| enalapril maleate | 1% |
|---|---|
| beeswax | 2-5% |
| propyl gallate | 0.01% |
| MCT or soybean oil | ad 100% |

Comparative Example 5

| enalapril maleate | 1% |
|---|---|
| silica dioxide | 2-6% |
| bentonite | 6.5% |
| propyl gallate | 0.01% |
| MCT or soybean oil | ad 100% |

The formulations of Examples 1 to 7 were stable formulations, whereas the formulations according to Comparative Examples 1 to 5 were not physically stable according to visual evidence of sedimentation volume. (example of preliminary results for 3 months stability in room temperature, 4°C and 30°C/65%(relative humidity = RH). The results are presented in the following Table 1:

## Table 1

Name of product: ENL drops (4 mg/ml)- Soybean, formulation according to Comparative Example 1

Storage time: 3m

| No. | Name of Test | Specification | Method | 30°C/65%RH | RT | 4°C |
|---|---|---|---|---|---|---|
| 1 | Description: a) Appearance b) Odor | a) oily viscous suspension b) Characteristic | a) Visual b) Olfactory | a) oily viscous suspension b) Characteristic | a) oily viscous suspension b) Characteristic | a) oily viscous suspension b) Characteristic |
| 2 | Assay (mg/ml), Range(%):95.0-105.0 | 4.00 (3.60-4.40) | 436D01 | 2.66* | 2.72** | 0.86** |
| 3 | Related substanses (%): DKP Enalaprilate Any other Total(%) | NMT 0.9 NMT 2.6 NMT 0.5 NMT 5.0 | 435D01 | 1.3* 0.4 0.7% (RRT=0.65) 2.4 | 0.7 <RL <RL 0.7 | 0.1 <RL <RL 0.1 |
| 4 | Dissolution (%) | NLT 70% in 45min | 436D02 | | | |
| 5 | Viscosity (cP): | NLT 1000 | 436D03 | | | |
| 6 | Peroxide value (meqO2/kg) | Soybean - NMT 20 | EP 2.5.5 | | | |
| 7 | Suspendability (shakes) | NMT 10 | P-GEN-73 | PASS | PASS | PASS |
| 8 | Sedimentation volume (%) | NLT 50 | P-RND-31 | 50 | 53 | 100 |

Remarks: Closed under nitrogen conditions! :*- out of specification (high RSD% due to non homogenic sample)

Determination of sedimentation volume:

This method is intended for estimation of degree of Flocculation when two layers are observed. The Sedimentation Volume, F, is the ratio of the equilibrium volume of the sediment, $V_u$, to the total volume of the suspension, $V_0$.

When F=1, no sediment is apparent even though the system is flocculated. This is the ideal suspension for, under these conditions, no sedimentation will occur. Caking will also be absent.

The test was performed with two separate bottles. The bottles should stand upright and should not be disturbed before the examination. The total height ($V_0$) of the suspension in each bottle was measured with the caliper. The sedimentation height ($V_u$) was measured with the caliper. The sedimentation volume (F) is calculated according to the following formula:

$$F\,(\%) \; = \; \frac{Vu}{Vo} \times 100$$

Suspendability test:

This test is intended to determine, under defined conditions, the suspendability of suspensions, i.e., the ability of a suspension to be re-dispersed by an appropriate procedure such as shaking.

Three separate containers were tested. The sample was brought to the temperature of 20-25°C. The container to be tested was carefully held. The container was shaken vigorously many times. The container was opened and its content was poured into a beaker. It was noted whether there is any precipitate left on the bottom of the container.

The results were determined as follows:

- If there is no precipitate left on the bottom of the container and the suspension appears to be homogenous, the sample has passed the test.

Report - "Pass".

- If there is a precipitate left on the bottom of the container and/or the suspension does not appear to be homogenous, the sample has not passed the test.

Report - "Not Pass".

- In case when all three samples have passed the test report the results.

Easy re-dispersion of the settled particles that form flock and not aggregates. Chemical stability of the active ingredient was evidenced by validated HPLC techniques based on USP/EP monographs.

Further, the method for preparing the stable liquid oily ready-to-use formulation according to the present invention is described in the following exemplary method.

**Example 8**

Assay on impurities and degradation products and dissolution test

The following formulations were tested:

Omeprazole formulation #300892 contains %w/w:
Omeprazole magnesium - 5%
Bentonite - 6.5%
Silica dioxide - 6%
Propyl gallate - 0.01 %
MCT - q.s.

Method of preparation of the formulation:

Mixing MCT oil with propyl gallate, adding bentonite and silica dioxide with stirring, when homogeneity is reached, adding the omeprazole and mixing until final homogeneity

For Assay and Impurities and Degradation Products Determination test 1 g of sample are weighted into 200 ml volumetric flask, then 20 ml of methanol are added and the sample is sonicated for 10 minutes, then it is diluted to volume with methanol, further on the above solution is diluted 5.0:50 with the same solvent and is filtered through 0.45$\mu$ PTFE filter prior to injection. Standard solution is 0.05 mg/ml of Omeprazole Mg.

The test performs with Betasil C8, 5 $\mu$, 4.6*150 mm column, at 30°C; eluent is 0.01 M phosphate buffer pH 7.6/ acetonitrile (65:35 v/v).

Enalapril formulation #291291 contains %w/w:

Enalapril maleate - 1%
Glyceryl distearate - 3%
Propyl gallate - 0.02%
Sucralose - 0.1%
Flavour - 0.2%
Soybean oil - q.s.

Method of preparation of the formulation:

Mixing MCT oil with propyl gallate, sucralose and flavour, adding premelted glyceryl distearate and mixing until homogeneity is reached, adding enelapril maleate and mixing until homogeneity.

For Assay and Impurities and Degradation Products Determination test 4 g of sample are weighted into 100 ml volumetric flask, then 20 ml of methanol are added and the sam- ple is shaked for 30 minutes at ambient temperature, then it is diluted to volume with 0.05M phosphate buffer pH

6.8 and is filtered through 0.45μ GHP filter prior to injection. Standard solution for assay level is 0.4 mg/ml and for IDD level is 0.002 mg/ml of Enalapril Maleate in 0.01 M phosphate buffer solution pH 2.2.

The test performs with Acclaim C8, 5 μ, 4.6*250 mm column, at 50°C; eluent is 0.01 M phosphate buffer pH 2.2/ acetonitrile (75:25 v/v).

The dissolution test is performed at followed conditions: 0.25% Sodium lauryl sulphate (so called "SLS"), 500 ml, 75 rpm (Apparatus 2 - paddle). The results are obtained by HPLC method. The dissolution sample is filtered prior to HPLC analysis.

The dissolution test has been carried out according to European pharmacopeia 6th edition 2010, Chapter 2.9.3.

The following results were obtained:

| Tests | Enalapril suspension #291291 | | Omeprazole suspension #300892 | |
|---|---|---|---|---|
| | Specifications (Enalapril) | Results - release | Specifications (Omeprazole) | Results - release |
| Assay (%) * | 95.0-105.0% | 99.7 | 95.0-105.0% | 98.2 |
| Related substances (total) (%) ** | ***NMT 5.0 | 0.3 | NMT 2.0 | 0.2 |

- *Assay (%): Quantitative determination of actual concentration of API (active pharmaceutical ingredient) in terms of % of the expected amount.

- ** Related substances (total)(%) : Quantitative determination of the impurities and degredation products reformed from the API, in terms of % of the API.

- *** NMT : Not more than

**Example 9**

Stability tests of Enalapril suspensions

The following enalapril formulations were prepared:

| Formulation 1 : | Enalapril maleate | 1% |
|---|---|---|
| | Glyceryl distearate | 3% |
| | Sweetener | 0.1% |
| | Flavour | 0.2% |

(continued)

| | | |
|---|---|---|
| | Antioxidant (propyl gallate) | 0.02% |
| | Soybean oil | ad 100% |
| Formulation 2: | Enalapril maleate | 1% |
| | Glyceryl distearate | 3% |
| | Sweetener | 0.1 % |
| | Flavour | 0.2% |
| | Antioxidant (propyl gallate) | 0.02% |
| | Oleoyl macrogolglycerides | 0.5%, 1.0%, 2.0%, 5.0% or 10.0% (in soybean 0.5%, 1.0%,oil) |
| | Soybean oil | ad 100% |

The above formulations were prepared according to the general description of the above method example. The formulations were tested for the following parameters:

a) Test for stability

The stability of the formulations with different concentrations of the emulsifier were tested at the freshly prepared formulation ($T_o$) and after a three months' storage at 30°C/65% RH and at RT and 4°C.
The results are given in the following Table 2.

Table 2:

| | | | | | | |
|---|---|---|---|---|---|---|
| ***Stability results of Enalapril suspension with different concentrations of the emulsifier*** | | | | | | |
| | **To** | | | | | |
| **%** | **0% Emulsifier** | **0.5% Emulsifier** | **1.0% Emulsifier** | **2.0% Emulsifier** | **5.0% Emulsifier** | **10% Emulsifier** |
| **Enalapril** | 86.1 | 104 | 105.0 | 102.0 | 99.4 | 93.2 |
| **Limits of Assay** | 95 | 95 | 95 | 95 | 95 | 95 |
| **Limits of Total IDDs** | 5 | 5 | 5 | 5 | 5 | 5 |
| **Enalaprilat** | 0.1 | 0.2 | 0.2 | 0.2 | 0.3 | 0.3 |
| **DKP** | 0 | 0.1 | 0.1 | 0.1 | 0.1 | 0.1 |
| **Total Impurities** | 0.3 | 0.6 | 0.6 | 0.7 | 1 | 1 |
| | **3 months** | | | | | |
| **%** | **0% Emulsifier** | **0.5% Emulsifier** | **1.0% Emulsifier** | **2.0% Emulsifier** | **5.0% Emulsifier** | **10% Emulsifier-1m** |
| **Enalapril** | 98.8 | 98.7 | 96.2 | 92.7 | 96.7 | 91.6 |
| **Limits of Assay** | 95 | 95 | 95 | 95 | 95 | 95 |
| **Limits of Total IDDs** | 5 | 5 | 5 | 5 | 5 | 5 |
| **Enalaprilat** | 0 | 0.1 | 0.1 | 0.1 | 0.2 | 0.1 |
| **DKP** | 0.6 | 1.1 | 1.1 | 1.3 | 1.1 | 0.5 |

(continued)

| % | 3 months | | | | | |
|---|---|---|---|---|---|---|
| | 0% Emulsifier | 0.5% Emulsifier | 1.0% Emulsifier | 2.0% Emulsifier | 5.0% Emulsifier | 10% Emulsifier-1m |
| Total Impurities | 0.8 | 1.4 | 1.6 | 1.8 | 1.4 | 0.7 |

IDDS= Impurities of degredation products
DKP= diketone piperazine

The results are summarized in Figure 1.

b) Test for physical stability and uniformity of the formulation.

The physical stability of the following formulation has been tested.

| Ingredient | Function | mg/ml |
|---|---|---|
| Enalapril Maleate | Active agent | 10.00 |
| Glycerol distearate | Lubricant | 30.00 |
| Sucralose | Sweetening agent | 2.00 |
| Orange Powder | Flavour agent | 2.00 |
| Propyl Gallate | Antioxidant | 0.20 |
| Soybean oil | Vehicle | 880.50 |

In order to verify the uniformity of dose of the obtained stable formulation, uniformity of content test was performed according to pharmacopoeias guidance. 0.6ml dose (corresponding to 6mg API) was sampled 10 times and assay test was performed on each dose to assure the dose was homogenous. The Acceptance Value (AV) was calculated according to pharmacopoeias guidance.

In order to improve AV, several parameters were changed:

a) prior dispersing of the powders in the formulation

b) adding powders at different temperatures

c) Duration and power of homogenizer

d) Order of addition of different phases (liquid/powders)

e) Particle size of the API and other powders in the formulations.

Duplicates of the formulation were placed at 30°C/65%RH, RT (room temperature), 4°C, and freezer (-20°C) (cycles of 24 hours of freeze and thaw). After 10 days of stability at various conditions the physical parameters (as specified at Table 3) was measured and summarized.

The results are summarized in the following Table 3.

Table 3:

| | | RT(Room Temperature) (2 bot) | 30°C/65% RH (4 bot.) | | Freeze/Thaw (2 bot.) | 4°C (2 bot.) |
|---|---|---|---|---|---|---|
| 1 | **Sedimentation volume** | | **Without shaking** | **With shaking** | F=98.4% | F=100% |
| | | F*=93.7% | F=83.0% | NA** | | |
| 2 | **Appearance Before shaking** | The sediment looks homogeneous with thin clear (40mm) layer above. There is no cake, the color and odor are as specified | The sediment looks homogeneous with thick clear (1cm) layer above. There is no cake, the color and odor are as specified. | Homogenous suspension, without cake, the color and odor are specified | The sediment looks homogeneous with very thin clear layer above. There is no cake, the color and odor are as specified. | Immediately after removal: Homogenous very viscous, not flowing suspension After 15 min: Viscous hardly flowing suspension, looks like jaily, without cake. |
| 3 | **Suspendability** | Conforms Without lumps, smooth, freely flowing suspension. Thin layer remained on the bottle. | Conforms Without lumps, smooth, freely flowing suspension. No difference between the two groups. | | Conforms Without lumps, freely flowing suspension | Conforms Without lumps, freely flowing suspension |
| 4 | **Appearance After shaking** | Homogenous suspension. Velvety texture. | Similar to RT conditions | | Similar to RT conditions | Without lumps, freely flowing suspension |
| 5 | **Visual viscosity** | Viscous, liquid, freely flowing and suspended. | Similar to RT conditions | | Similar to RT conditions. | Similar to RT conditions. |

*F - Sedimentation volume (%)

The above stability results show that the viscosity of the suspension is altered at 30°C/65% RH. Therefore, a storage temperature of not more than 25°C is suitable.

It has, moreover, been found that the formulation is stable at room temperature conditions for 30 days of use. In a corresponding Assay following results have been obtained. Release: 104%, after 30 days 97%. The total impurities were also within the specification.

c) Flavours adaption: powder vs. liquid flavours

The above formulation 1 has been tested for its adaption to flavours. Several preparation methods were compared in order to achieve uniformity of the dose. The batches tested:

#241001 - without prewetting the powders, prepared mostly by blender.

#091103 - As #241001 but with prewetting the powders.

#141201 - As #091103 with smaller particle size of active material.

#281201 - with prewetting the powders, prepared in mixer vacuum mostly by homogenizer.

The results are summarized in Table 4.

Table 4:

| Test | Conditions | Specs | Sampling | Results | | | |
|------|-----------|-------|----------|---------|---|---|---|
| | | | | #241001 *** (40mg/ 4ml) | #091103 | #141201 | #281201 |
| Homogeneity (glass) 0.6ml | After final mixing by Heidolph(1-2 hours after homogenization) | 6.0mg/0.6ml | A1 (top1) | 41.1 | 5.6 | 5.4 | 5.9 |
| | | | A2 (top2) | | 5.9 | 5.3 | 5.9 |
| | | | B (middle) | 41 | 6.6 | 5.5 | 5.8 |
| | | | C1 (bottom 1) | 41.1 | 6.5 | 5.4 | 5.9 |
| | | | C2 (Bottom 2) | | 6 | 5.3 | 5.9 |
| Test | Dose | Specs | Batch No. | #241001 | #091103 | #141201 | #281201 |
| Uniformity of content (delivered dose) | 0.6ml | 6.0mg/dose (95.0-105.0%) | | 116%* | 91.5%* | 90.0%* | 96.7% |
| | | | | AV**=58* | AV = 28.4* | AV=14.5 | AV=5.04 |
| Out of specification **AV - Acceptance value acc. to pharmacopeias specifications of Uniformity of content *** The sample dose was 4ml (and not 0.6ml as in the others samples, the concentration remained the same) | | | | | | | |

As it can be seen, the Batch No. 281201 is homogenous and uniform.

Different flavours, liquid and solid, were tested in the formulation. Liquid flavours contained too many impurities, destabilized the formulation physically, and chemically, as it appeared in the stability studies. In addition, liquid flavours oxidised the formulation in such a way that the antioxidant were not efficient and there was a need to increase its concentration dramatically and therefore to exceed the recommended levels. We found that solid flavours (powders) were more stable in the formulation.

The above experiments show that powder flavours are more suitable for the enalapril suspension due to better stability and less interaction.

The results of the above experiments lead to the following conclusion as regards the stability and uniformity of an enalapril suspension:

1. The obtained formulation is stable at room temperature for at least 6 month and is should be stable at refrigerator (4°C).

2. The obtained formulation is stable for the proposed period of use (30 days) at room temperature, compared to refrigerator at existing extemporaneous preparation.

3. The technology of the preparation is highly important in order to obtain uniform formulation at low doses.

4. As long as the particle size of the API and other powder ingredients is smaller, than the uniformity/homogeneity of the formulation is better.

5. When stored at room temperature or lower, the formulation is physically stable. At temperatures above 25°C, the physical stability will be altered.

**Example 10**

Stability tests of Amiodarone Suspensions

| Formulation 1: | Amiodarone HCl | 10% |
| --- | --- | --- |
| | Glyceryl distearat | 3% |
| | Span 60 | 1.03% - 2.55% |
| | Tween 80 | 0.97% - 2.45% |
| | Propyl gallate | 0.02% (in soybean oil) |
| | Aerosil | 0-2% |
| | Sweetener | 0.1 % |
| | Flavour | 0.2% |
| | Medium chain triglycerides | ad 100% |

Formulation 2:  same formulation as Formulation 1 except that 0.01% propyl gallate (MCT) was used instead of 0.02% propyl gallate in soybean oil.

The formulations were prepared as follows:

a) Heating the oily vehicle under mild stirring.

b) Dissolving antioxidant.

c) Dissolving/melting the thickening/suspending agent until clear solution is obtained and cooling the solution to room temperature.

d) Dispersing the powder components of the formulation (API, and optionally sweetener and/or flavouring and or colouring agents.

e) Adding d) optionally under vacuum conditions to c) or vice-versa while vigorous stirring or homogenizer.

f) Optionally adding sweetener and/or flavouring and/or colouring agent and mixing.

g) Completing volume with oily vehicle to the desired amount and mixing.

The formulation was tested as follows:

- Preliminary stability -2m at 40°C/65%RH, 30°C/65%RH and RT

  - Glycerol distearate 3%, Propyl gallate 0.02% (in Soybean oil)

  - Glycerol distearate 3%, Propyl gallate 0.01 % (in MCT)

- Dissolution adjustment:

  - With emulsifiers (Tween, Span, Oleoyl macrogolglycerides, Caprylo caproyl Macrogolglycerides) in different combinations in Soybean oil.

  - With emulsifiers (Tween, Span, Oleoyl macrogolglycerides, Caprylocaproyl Macragolglycerides) in different combinations in MCT.

  - Different total concentrations of the emulsifiers (Tween 80, Span 60) - 1.0%, 1.5%, 2.0%, 2.5% or 5.0%

- Preliminary stability for 6m at 40°C/75%RH, 30°C/65%RH and RT

- Glicerol distearate 3.0%, Span 60/Tween80 2.0%, propyl gallate 0.01%, colloidal silica dioxide 0.5%, sweetener 0.1%, Flavour 0.2% (in MCT).

- Viscosity adjustment:

- With/without colloidal silica dioxide + Emulsifiers (in Soybean oil)

- With/without colloidal silica dioxide + Emulsifiers (in MCT)

The results are summarized in the following Tables 5 to 7.

Table 5:

| | Physical stability results | | | | |
|---|---|---|---|---|---|
| No. | Name of Test | RT | 40c/75%RH RT (B) | 40c/75%RH 4C (A) | (-)20C / RT |
| 1 | Description:<br>a) Appearance<br>b) Odor | a) oily viscous suspension<br>b)Characteristic | a) oily viscous suspension<br>b)Characteristic | a) oily viscous suspension<br>b)Characteristic | a) oily viscous suspension<br>b)Characteristic |
| 2 | Sedimentation volume (%) | 94 | 93 | 100 | 93 |
| 3 | Suspendability (shakes) | Not conforms. No caking and no lumps but the suspension is not flowing out of the bottle.<br>A thick layer remained on the bottle. | Conforms Without lumps, smooth, freely flowing suspension. Only thin layer remained on the bottle. | Not conforms. No caking and no lumps but the suspension is not flowing out of the bottle.<br>A thick layer remained on the bottle. | No caking and no aggregates but the suspension stayed on the bottle walls |
| 4 | Viscosity (cP): | 3773 | 1140 | 1768 | 2985 |

Table 6: - $T_0$ (Release)

| | Preliminary stability (3 months period) results | | |
|---|---|---|---|
| No. | Name of Test | Specification | Release |
| 1 | Description:<br>a) Appearance<br>b) Odor | a) oily viscous suspension<br>b)Orange | a) oily viscous suspension<br>b)Orange |
| 2 | Identification of Amiodarone | 1) HPLC<br>2) UV | 1) conforms<br>2) conforms |
| 3 | Assay (mg/ml), Range(%):95.0-105.0 | 100.0<br>(95.0 - 105.0) | 100.2 |
| 4 | Related substanses (%)(BP):<br>Any individual impurity<br>Total impurities | NMT 0.2<br>NMT 0.5 | <RL** |
| 5 | Propyl gallate content (mg/ml) Range: 90.0 - 110.0% | 0.10<br>0.09 - 0.11 | 0.091* |
| 6 | Dissolution and particle size consistency by dissolution (%) | NLT 70% in 45min | 100% |

(continued)

| No. | Name of Test | Specification | Release |
|---|---|---|---|
| | Preliminary stability (3 months period) results | | |
| 7 | Weight per mililiter (g/ml): | Monitoring | 0.99 |
| 8 | viscosity (cP): | NLT 1000 | 7077 |
| 9 | Peroxide value (meqO2/kg) | NMT2 | 0.9 |
| 10 | Suspendability (shakes) | NMT 10 | conforms |

Table 7

| No. | Name of Test | Specification | 40°C/75%RH (Under Nitrogen conditions) | 40°C/75%RH** | RT (Under Nitrogen conditions) |
|---|---|---|---|---|---|
| | 3m Stability | | | | |
| 1 | Description: a) Appearance b) Odor | a) oily viscous suspension b)Orange | a) Conforms b) Does not conform | a) Conforms b) Does not conform | a) Conforms b) Does not conform |
| 2 | Assay (mg/ml), Range(%): 95.0-105.0 | 100.0 (95.0-105.0) | 96.8 | 97.1 | 101.4 |
| 3 | Related substances (%) (BP): Any individual impurity Total impurities | NMT 0.2 NMT 0.5 | 0.2 total 0.2 | 0.2 total 0.2 | <DL |
| 4 | Propyl gallate content (mg/ml) Range: 90.0 - 110.0% | 0.10 0.090-0.11 | | | |
| 5 | Dissolution and particle size consistency by dissolution (%) | NLT 70% in 45min | | | |
| 6 | Viscosity (cP): | NLT 1000 | | | |
| 7 | Peroxide value (meqO2/kg) | NMT2 | | | |
| 8 | Sedimentation volume (%) | NLT 50 | 36 | | 100 |
| 9 | Suspendability (shakes) | NMT 10 | pass | pass | pass |

The above test demonstrated that Amiodarone is stable at accelerated conditions (40°C/75% RH) for three months. Due to the lipophilic nature of the active material there is a need for emulsifiers to ensure dissolution. It has further been found this formulation has a thixotropy characteristic, i.e. it is thick and getting flow after shaking. Heat, even for a short period of time, destroys the thixotropy characteristics of the formulation. The formulation looses its viscosity permanently after heating even for a short term. Moreover, the formulation's odor is changing.

**Example 11**

Monolayers of differentiated Caco-2 (epithelial colorectal adenocarcinoma cells) cells show morphological and biochemical similarity to normal intestinal enterocytes, and they develop tight effective junctions. Thus, Caco-2 cell monolayer is considered an established model to investigate the mechanisms involved in oral absorption including the effect of the P-gp pump. The P-gp expression of the actual Caco-2 monolayer was evidenced (data not shown) using the validated monoclonal antibody C219 technique. The following oily enalapril formulations of the invention were tested:

Protocol of the Experiment

Caco-2 monolayer were grown in Dulbecco's modified Eagle's medium supplemented with 10% fetal bovine serum, 1% L-glutamine, 1% nonessential amino acids, and 5% antibiotic-antimycotic solution at 37°C in humidified air, 5% $CO_2$ atmosphere. The culture medium was replaced every 96 h. The uptake studies were conducted with monolayers (passages 73 to 79) in wells of 2 $cm^2$ which reach confluency following 4 days of incubation in culture. Prior to the experiments, the culture medium was replaced with transport medium and cell monolayers were subsequently equilibrated for 30 min at 37°C before the uptake study. The transport medium was Hanks buffer composed of 136.89 mM NaCl, 5.36 mM KCl, 0.34 mM Na2HPO4, 0.44 mM KH2PO4, 0.41 mM MgSO4 7H2O, 19.45 mM glucose, 1.26 mM CaCl2, 0.49 mM MgCl2 6H2O, 4.17 mM NaHCO3, 10 mM HEPES, and the pH was adjusted to 7.4. The cell monolayerswere washed twice with 1 ml Hanks solution and then combined with the transport medium (1 ml) which contained 25 μg/ml of enalapril maleate (provided by CTS, RLB: 802871, E65 mean diameter smaller that 100μm)dissolved in an appropriate solvent preferably in water, or aliquot of enalapril tablet CTS (Xanef® 20 mg tablets. Batch number, 30056.03.00 Expiry date 0.1-2011) or as enalapril CTS oil formulation (ENL drops 10 mg/ml lot. 030301). In addition, the influence of increasing concentrations of intestinal juice (IJ) was also tested. Xanef® comprises the following excipients: sodium hydrogen carbonate, lactose monohydrate, corn starch, pregelatinized corn starch, magnesium stearate, iron (III) oxide E172 (colorant), iron (II) hydroxide-oxide.

The following formulation was tested: CTS-COCO2-no 1

| Ingredient | Function | mg/ml |
|---|---|---|
| Enalapril Maleate | Active agent | 10.00 |
| Glycerol distearate | Lubricant | 30.00 |
| Sucralose | Sweetening agent | 1.00 |
| Tutti Frutti | Flavour agent | 2.00 |
| Propyl Gallate | Antioxidant | 0.20 |
| Soybean oil | Vehicle | Ad 100 ml |

Samples preparation

a) Enalapril tablet processing:

A tablet of enalapril maleate was grounded and sieved through a sieve of 366 μm aperture. The powder (10mg equivalent to 1mg enalapril maleate) was weighted dispersed and fully dissolved in 1 ml Hanks buffer.25μl from this solution were placed in Caco-2 wells with 975μl of Hanks buffer resulting in a final concentration of 25 μg/ml of enalapril maleate.

b) CTS oil formulation processing:

A 100 μl of parent CTS oil formulation was withdrawn and diluted with soybean oil USP to give 1 mg/ml of enalapril maleate.

A 25 μl of the diluted oil solution was placed into the transport medium to give a 25 μg/ml of enalapril maleate in each well (dilution 1:40).

c) Various CTS formulations+ 0.1 or 0.01 % IJ processing:

These experiments was conducted under similar experimental conditions as described in section b. except;

the dilution medium containing 0.1%; 0.01 % of intestinal juice.

The final objective of this study was to design and partially validate a Caco-2 cells method by which usually water soluble formulations are tested, to be able to teach on the potential absorption of CTS oil formulation following oral administration to humans as compared to standard tablets of enalapril.

All the uptake experiments were performed over 3 hours at 37°C. Following incubation the transport medium was collected in clean tubes stored at -80 ° C up to the quantitative determination of the enalapril levels by HPLC (The HPLC method was provided by CTS). Regarding the cell monolayer in the wells, 1 ml of 0.2% SDS was added to each well to elicit lysis of the cell monolayer. The lysate were collected in a clean tube and enalapril was determined by CTS HPLC technique.

Statistical tests

Analysis was determined with the Tukey-Kramer multiple comparisons test calculated by InStat software (version 3.01). The level of significance was corrected using a post test analysis. Statistical significance was set with one * for $p < 0.05$ and with *** for $p < 0.001$ while values are presented as mean $\pm$ S.D.

Results and Discussion:

In preliminary studies, the incubation time of the various CTS formulations with the Caco-2 cells was investigated and the data indicated that no difference could be detected when the formulations were incubated over 1 h only and 3 hours were needed to elicit a significant enalapril uptake by the Caco-2 cells. Indeed, enalapril residual concentration in the wells diminished by more than 50% in the presence of Caco-2 cells from the apical side when dissolved in Hanks buffer or released from the tablet whereas about 80% of the initial enalapril concentration remained in the well and only 20% of the enalapril was up taken by the Caco-2 cells following incubation over 3 h. This probably was due to lack of partition of the drug from the oil towards the Hank buffer solution because the oil solution did not disperse well into the aqueous phase (Fig. 2).

This was confirmed by the data presented in Fig. 3 showing the respective enalapril uptake by the Caco-2 cell monolayer in the presence of increasing concentrations of rat intestinal juice. It should be emphasized that higher concentrations of intestinal juice were toxic to the cells. Nevertheless, the highest drug uptakes were achieved by the enalapril oil formulation in 0.1 % intestinal juice indicating that the dispersion and self-emulsification extent of the CTS oil formulation is a critical parameter in terms of drug uptake in the set up of the Caco-2 technique. Furthermore, it should be noted that the enalapril maleate uptake from CTS tablet was not significantly improved by the addition of intestinal juice at the highest concentrations (Table1 & Fig. 4) suggesting that the enhanced cell uptake in the case of CTS oil formulation was rather due to a self emulsification process mediated by the presence of the intestinal juice that accelerated the release of enalapril from the formulation and not to an intrinsic enhancement penetration effect of the enalapril in the Caco-2 cells (Fig.4).

The apparent permeability ($P_{app}$, cm/s) was calculated according to the following equation described by Schrickx and Fink-Gremmels (Schrickx, J. and Fink-Gremmels, J. P-glycoprotein-mediated transport of oxytetracycline in the Caco-2 cell model. J. Vet Pharmacol. Therap. 30: 25-31 (2007)).

$$P_{app} = \frac{Q}{A \times C_o \times t} \qquad \text{Equation 1}$$

Where Q is the total amount of permeated drug throughout the incubation time period ($\mu g \, t^{-1}$), A is the diffusion area (2 cm$^2$), $C_o$ is the initial concentration (25$\mu$g/ml) and t is the incubation time (3 hours=10800 seconds) in seconds.

Statistical analysis of the apparent permeability ($P_{app}$) values calculated from equation 1 (Table 1), clearly indicated that there was no significant difference between the enalapril aqueous formulation (25.09$\pm$0.50 10$^{-6}$, cm/s), CTS oil formulation + 0.1 % intestinal juice (21.85 $\pm$ 2.44 10$^{-6}$cm/s) and the Tablet formulation (20.89 $\pm$ 2.93 10$^{-}$cm/s).

**Table-8: The apparent permeability ($P_{app}$,cm/s) of the various CTS formulations.**

| Formulation | $P_{app}X10^6$ (cm/s) $\pm$ s.d | |
|---|---|---|
| ENL-solution | 25.09 | $\pm$2.93 |
| ENL+IJ 0.1% | 25.20 | $\pm$4.81 (NS) |
| CTS | 10.76 | $\pm$1.50 (***) |

(continued)

| Formulation | $P_{app}X10^6$ (cm/s) $\pm$ s.d | |
|---|---|---|
| CTS + 0.1 % IJ | 21.85 | $\pm$2.44 (NS) |
| Tablet | 20.89 | $\pm$2.93 (NS) |
| Tablet + 0.1% IJ | 15.65 | $\pm$4.81 (NS) |

Conclusions

It can be concluded from the overall results that the CTS oil formulation elicits a similar enalapril uptake compared to the tablet and aqueous solution of enalapril maleate under experimental conditions mimicking the normal physiological GI conditions in Caco-2 monolayer cells.

**Claims**

1. A stable liquid oily ready-to-use formulation, comprising:

   (i) an active pharmaceutical ingredient, which has hydrophobic and/or lipophilic properties and/or which exhibits stability problems in aqueous environments,
   (ii) an oily vehicle, in which the active pharmaceutical ingredient is dissolved or dispersed, and which is selected from vegetable oils, synthetic oils, fatty acids or combinations thereof; and optionally one or more of a thickening/suspending agent, an antioxidant, a preservative, a flocculating agent, a surface stabilising agent, a sweetener, a flavouring agent, an emulsifier, and a colouring agent, or combinations thereof.

2. The formulation of claim 1, wherein the vegetable oil is selected from wheat germ oil, soybean oil, olive oil, arachis oil, corn oil, cottonseed oil, linseed oil, coconut oil, rapeseed oil, borage seed oil, apricot kernel oil, peanut oil, sunflower oil, sesame oil, safflower oil, sweet almond oil or combinations thereof.

3. The formulation of claim 1 or 2, wherein the synthetic oil is selected from medium chain triglycerides (MCT), propylene glycol dicaprylocaprate, glyceryl behenate, glyceryl monolinoleate, glyceryl oleate, glyceryl caprylate/caprate, glyceryl cocoate, butylene glycol dicaprylate/caprate, coco caprylate/caprate, hydrogenated vegetable oils, refined vegetable oils, glyceryl laurate, glyceryl myristate or combinations thereof.

4. The formulation any of claims 1 to 3, wherein the fatty acid is selected from $C_8$-, $C_9$-, $C_{10}$-, $C_{11}$-, $C_{12}$-, $C_{13}$-, $C_{14}$-, $C_{15}$-, $C_{16}$-, $C_{17}$-, $C_{18}$-, $C_{19}$-, $C_{20}$-, $C_{21}$-, $C_{22}$- fatty acids or combinations thereof.

5. The formulation of any of claims 1 to 4, wherein the thickening/suspending agent is selected from polysaccharides, fumed silica dioxide, acacia, pectin, kaolin, bentonite, cetyl alcohol, beeswax, glyceryl palmitostearate, cetostearyl alcohol, and magnesium albuminium silicates, or combinations thereof.

6. The formulation of any of claims 1 to 5, wherein the antioxidant/preservative is elected from propyl gallate, butylated hydroxytoluene (BHT), butylated hydroxyanisole (BHA) or combinations thereof.

7. The formulation of any of claims 1 to 6, wherein the surface stabilising agent is selected from kaolin, magnesium aluminium silicates, glycerine, and bentonite, or combinations thereof.

8. The formulation of any of claims 1 to 7, wherein the oily vehicle is soybean oil or a MCT.

9. The formulation of any of claims 1 to 8, wherein the thickening/suspending agent is beeswax, glyceryl palmitostearate or a combination thereof.

10. The formulation of any of claims 1 to 9, wherein the antioxidant/preservative is BHT and/or propyl gallate.

11. The formulation of any of claims 1 to 10, wherein the active pharmaceutical ingredient is selected from enalapril maleate, bisacodyl, captopril, omeprazole, domperidone, famotidine, gabapentin, granisetron HCl, itraconazole,

**EP 2 674 152 A1**

ketoconazole, lansoprazole, levodopa/carbidopa, losartan potassium, ondansetron HCl, amiodarone HCl, rifampicin, sildenafil citrate, dexa- dexamethasone, verapamil, spirospironolactone, tacrolimus, tramadol HCl, tramadol-paracetamol, baclofene, terbinafine HCl, or spironolactone-hydrochlorothiazide.

12. The formulation of any of claims 1 to 11, wherein the active pharmaceutical agent is enalapril maleate or omeprazole and the salts of thereof.

13. The formulation of any of claims 1 to 11, comprising a ratio of 0.1-20% of the active pharmaceutical ingredient to the oily vehicle.

14. The formulation according to any of claims 1 to 12 having a dissolution rate according to general EP specifications of not less than 70% in 45 min.

15. The formulation according to any of claims 1 to 15, in the form of an oral, topical or parenteral formulation.

16. Use of a stable liquid oily ready-to-use formulation according to any of claims 1 to 15 for the manufacture of a medicament for the medical treatment of a patient group selected from (i) paediatric patients, (ii) elderly patients, (iii) patients suffering from dysphagia, or (iv) patients requiring medication via nasogastric or gastrostomy tubes.

17. The stable liquid oily ready-to-use formulation according to any of claims 1 to 15 for use in the medical treatment of a patient group selected from (i) paediatric patients, (ii) elderly patients, (iii) patients suffering from dysphagia, or (iv) patients requiring medication via nasogastric or gastrostomy tubes.

18. A method for preparing a stable liquid oily ready-to-use formulation according to any of claims 1 to 15, comprising the following steps:

(a) Heating the oily vehicle under mild stirring,
(b) Dissolving the antioxidant,
(c) Dissolving or melting the thickening/suspending agent and optionally emulsifying agent(s), until a clear solution is obtained, and cooling the solution to room temperature,
(d) Optionally adding further thickening/suspending agent,
(e) Adding and optionally dispersing the active pharmaceutical ingredient,
(f) Optionally adding the sweetener, flavouring and/or colouring agents,
(g) Completing the volume with the oily vehicle to the desired amount, and
(h) Optionally performing homogenisation.

19. The method of claim 17, wherein the active pharmaceutical ingredient is granulated or encapsulated into microparticles prior to adding/dispersing the active pharmaceutical ingredient in the vehicle.

23

Figure 1

Figure 2

Figure 3

EP 2 674 152 A1

Figure 4

EP 2 674 152 A1

**EUROPEAN SEARCH REPORT**

Europäisches
Patentamt
European
Patent Office
Office européen
des brevets

Application Number

EP 13 18 3542

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 190 720 A1 (S A L V A T LAB SA [ES]) 27 March 2002 (2002-03-27) * page 2, paragraph 1 - paragraph 2 * * page 3, paragraph 15 * * examples 1-5 * * claim 1 * | 1-19 | INV. A61K9/10 A61K47/44 A61K31/4439 A61K31/401 |
| X | WO 2008/142231 A2 (TROPHOS [FR]; DROUOT CYRILLE [FR]; BERNA PATRICK [FR]) 27 November 2008 (2008-11-27) * page 3, line 1 - line 30 * * page 9; example 3 * * page 10, line 3 - line 9 * * claims 1,3,4 * | 1-19 | |
| X | US 2004/202696 A1 (YAMIN RINA [IL] ET AL) 14 October 2004 (2004-10-14) * examples 1-6 * | 1-19 | |
| X | WO 01/37834 A1 (DEBOECK ARTHUR M [US]; BIEST FRANCIS V D [BE]; BAUDIER PHILIPPE R [BE]) 31 May 2001 (2001-05-31) * examples 2,3,5-7 * * claims 1-2 * | 1-19 | TECHNICAL FIELDS SEARCHED (IPC) A61K |
| X | WO 2005/027880 A1 (NATCO PHARMA LTD [IN]; PODILI KHADGAPATHI [IN]; VENKAIAH CHOWDARY NANN) 31 March 2005 (2005-03-31) * examples 1,2,4,8 * * claim 1 * | 1-19 | |

The present search report has been drawn up for all claims

| Place of search | Date of completion of the search | Examiner |
|---|---|---|
| Munich | 8 October 2013 | Hedegaard, Anette |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 13 18 3542

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2013

| Patent document cited in search report | | | Publication date | Patent family member(s) | | | Publication date |
|---|---|---|---|---|---|---|---|
| EP | 1190720 | A1 | 27-03-2002 | AT | 262350 | T | 15-04-2004 |
| | | | | DE | 60009290 | D1 | 29-04-2004 |
| | | | | EP | 1190720 | A1 | 27-03-2002 |
| | | | | ES | 2153786 | A1 | 01-03-2001 |
| | | | | ES | 2215665 | T3 | 16-10-2004 |
| | | | | WO | 0076549 | A1 | 21-12-2000 |
| WO | 2008142231 | A2 | 27-11-2008 | AU | 2008252830 | A1 | 27-11-2008 |
| | | | | CA | 2680433 | A1 | 27-11-2008 |
| | | | | EP | 2124961 | A2 | 02-12-2009 |
| | | | | FR | 2914188 | A1 | 03-10-2008 |
| | | | | JP | 2010522729 | A | 08-07-2010 |
| | | | | NZ | 579632 | A | 25-05-2012 |
| | | | | RU | 2009139755 | A | 10-05-2011 |
| | | | | US | 2010099652 | A1 | 22-04-2010 |
| | | | | WO | 2008142231 | A2 | 27-11-2008 |
| | | | | ZA | 200906271 | A | 26-05-2010 |
| US | 2004202696 | A1 | 14-10-2004 | AU | 2004228786 | A1 | 21-10-2004 |
| | | | | CA | 2522206 | A1 | 21-10-2004 |
| | | | | EP | 1613279 | A1 | 11-01-2006 |
| | | | | JP | 2006522804 | A | 05-10-2006 |
| | | | | US | 2004202696 | A1 | 14-10-2004 |
| | | | | WO | 2004089337 | A1 | 21-10-2004 |
| | | | | ZA | 200508645 | A | 26-03-2008 |
| WO | 0137834 | A1 | 31-05-2001 | AU | 1545301 | A | 04-06-2001 |
| | | | | CA | 2392353 | A1 | 31-05-2001 |
| | | | | EP | 1259238 | A1 | 27-11-2002 |
| | | | | WO | 0137834 | A1 | 31-05-2001 |
| WO | 2005027880 | A1 | 31-03-2005 | AU | 2003272081 | A1 | 11-04-2005 |
| | | | | CA | 2540105 | A1 | 31-03-2005 |
| | | | | EP | 1663173 | A1 | 07-06-2006 |
| | | | | US | 2007196463 | A1 | 23-08-2007 |
| | | | | WO | 2005027880 | A1 | 31-03-2005 |

EPO FORM P0459

For more details about this annex : see Official Journal of the European Patent Office, No. 12/82

## REFERENCES CITED IN THE DESCRIPTION

**Non-patent literature cited in the description**

- **RIPPLEY, R. K. ; CONNOR, J. ; BOYLE, J. ; BRAD-STREET, T. E. ; HAND, E. ; LO, M-W. ; MURPHY, M. G.** *Biopharm. Drug Dispos.,* 2000, vol. 21, 339-344
- Drugs and the pharmaceutical sciences textbook. **REMINGTON.** Pharmaceutical emulsions and suspensions. 2000, vol. 105
- The pharmaceutical codex. 1994
- **SCHRICKX, J. ; FINK-GREMMELS.** J. P-glycoprotein-mediated transport of oxytetracycline in the Caco-2 cell model. *J. Vet Pharmacol. Therap.,* 2007, vol. 30, 25-31